# EUROPEAN PATENT APPLICATION

(11) **EP 0 657 469 A1**
(43) Date of publication of application: **14.06.1995**
(21) Application number: 94116350.3
(22) Date of filing: 17.10.1994
(51) Int. Cl.: C07K 14/705, C07K 14/81, C07K 16/28, C07K 2/00, G01N 33/68, G01N 33/53, C12N 9/64

(54) **L-Selectin cleavage site, methods of diagnosis and usage thereof**

(30) Priority: 26.10.1993 US 141672
(71) Applicant: BOEHRINGER INGELHEIM PHARMACEUTICALS INC., Ridgefield, Connecticut 06877-0368 (US)
(72) Inventor: Kishimoto, Takashi Kei, New Fairfield, CT 06812 (US); Kahn, Julius, Waterbury, CT 06708 (US)
(74) Representative: Laudien, Dieter, Dr.

(57) **Abstract**

The present invention discloses the cleavage sequence within L-selectin that becomes cleaved during neutrophil activation/stimulation. A previously unidentified approximately 6 kd L-selectin species, which remains associated with the activated/stimulated neutrophil, is also described. Based on these observation, the present invention provides methods of detecting L-selectin cleavage and methods of reducing the severity of biological responses associated with L-selectin cleavage.

SEQUENCE LISTING

(1) GENERAL INFORMATION:

(i) APPLICANT: KISHIMOTO, KEI

(ii) TITLE OF INVENTION: L-SELECTIN CLEAVAGE SITE, METHODS OF
DIAGNOSIS AND USAGE THEREOF

(iii) NUMBER OF SEQUENCES: 11

(iv) CORRESPONDENCE ADDRESS:
(A) ADDRESSEE: Sterne
(B) STREET: 1100 NEW YORK AVE
(C) CITY: WASHINGTON
(D) STATE: DC
(E) COUNTRY: USA
(F) ZIP: 20005

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 08/022,034
(B) FILING DATE: 25-FEB-1993

(vii) PRIOR APPLICATION DATA:
(A) APPLICATION NUMBER: US 08/026,063
(B) FILING DATE: 04-MAR-1993

(viii) ATTORNEY/AGENT INFORMATION:
(A) NAME: MILLMAN, ROBERT A
(B) REGISTRATION NUMBER: 5
(C) REFERENCE/DOCKET NUMBER: 1011.0870000

(ix) TELECOMMUNICATION INFORMATION:
(A) TELEPHONE: 202-371-2678

(2) INFORMATION FOR SEQ ID NO:1:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:1:

Lys Leu Asp Lys Ser Phe Ser Met Ile Lys Glu Gly Asp Tyr Asn
1               5                   10                  15

(2) INFORMATION FOR SEQ ID NO:2:

(i) SEQUENCE CHARACTERISTICS:
(A) LENGTH: 23 amino acids
(B) TYPE: amino acid
(D) TOPOLOGY: linear

(ii) MOLECULE TYPE: peptide

(xi) SEQUENCE DESCRIPTION: SEQ ID NO:2:

Pro Leu Phe Ile Pro Val Ala Val Met Val Thr Ala Phe Ser Gly Leu
1               5                   10                  15

Ala Phe Ile Ile Trp Leu Ala
            20

(2) INFORMATION FOR SEQ ID NO:3:

(i) SEQUENCE CHARACTERISTICS:
(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO:3:

Arg Arg Leu Lys Lys Gly Lys Lys Ser Lys Arg Ser Met Asn Asp Pro
1               5                   10                  15

Tyr

(2) INFORMATION FOR SEQ ID NO:4:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:4:

Cys Arg Arg Leu Lys Lys Gly Lys Lys Ser Lys Arg Ser Met Asn Asp
1               5                   10                  15

Pro Tyr Cys His
            20

(2) INFORMATION FOR SEQ ID NO:5:

(i) SEQUENCE CHARACTERISTICS:
(A) LENGTH: 22 amino acids
(B) TYPE: amino acid
(D) TOPOLOGY: linear

(ii) MOLECULE TYPE: peptide

(xi) SEQUENCE DESCRIPTION: SEQ ID NO:5:

Asn His Gln Lys Leu Asp Lys Ser Phe Ser Met Ile Lys Glu Gly Asp
1               5                   10                  15

Tyr Asn Pro Cys Cys His
            20

(2) INFORMATION FOR SEQ ID NO:6:

(i) SEQUENCE CHARACTERISTICS:
(A) LENGTH: 20 amino acids

(B) TYPE: amino acid
(D) TOPOLOGY: linear

(ii) MOLECULE TYPE: peptide

(xi) SEQUENCE DESCRIPTION: SEQ ID NO:6:

Cys Gln Lys Leu Asp Lys Ser Phe Ser Met Ile Lys Glu Gly Asp Tyr
1               5                   10                  15

Asn Pro Leu Phe
            20

(2) INFORMATION FOR SEQ ID NO:7:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:7:

Cys Gln Glu Thr Asn Arg Ser Phe Ser Lys Ile Lys Glu Gly Asp Tyr
1               5                   10                  15

Asn Pro Leu Phe
            20

(2) INFORMATION FOR SEQ ID NO:8:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:8:

Cys Gln Lys Thr Lys Arg Ser Phe Ser Lys Ile Lys Glu Gly Asp Tyr
1               5                   10                  15

Asn Pro Leu Phe
            20

(2) INFORMATION FOR SEQ ID NO:9:

     (i) SEQUENCE CHARACTERISTICS:
          (A) LENGTH: 12 amino acids
          (B) TYPE: amino acid
          (D) TOPOLOGY: linear

    (ii) MOLECULE TYPE: peptide

    (xi) SEQUENCE DESCRIPTION: SEQ ID NO:9:

Cys Glu Ala Pro Thr Glu Ser Asn Ile Pro Leu Val
1               5                   10

(2) INFORMATION FOR SEQ ID NO:10:

     (i) SEQUENCE CHARACTERISTICS:
          (A) LENGTH: 13 amino acids
          (B) TYPE: amino acid
          (D) TOPOLOGY: linear

    (ii) MOLECULE TYPE: peptide

    (xi) SEQUENCE DESCRIPTION: SEQ ID NO:10:

Cys Gln Ala Gly Pro Leu Thr Ile Gln Glu Ala Leu Thr
1               5                   10

(2) INFORMATION FOR SEQ ID NO:11:

     (i) SEQUENCE CHARACTERISTICS:
          (A) LENGTH: 35 amino acids
          (B) TYPE: amino acid
          (D) TOPOLOGY: linear

    (ii) MOLECULE TYPE: peptide

(xi) SEQUENCE DESCRIPTION: SEQ ID NO:11:

Pro Ile Cys Gln Lys Leu Asp Lys Ser Phe Ser Met Ile Lys Glu Gly
1               5                   10                  15

Asp Tyr Asn Pro Leu Phe Ile Pro Val Ala Val Met Val Thr Ala Phe
            20                  25                  30

Ser Gly Leu
        35

## Description

### Background of the Invention

### Field of the Invention

The present invention relates to the field of inflammation and anti-inflammation therapy. The present invention further relates to methods of modulating responses which are associated with L-selectin mediated neutrophil attachment and migration.

### Description of the Related Art

Leukocyte localization to sites of inflammation is a precisely coordinated multistep process involving distinct families of adhesion receptors and chemokines (Butcher, E. C., *Cell 67*:1033-1036 (1991), Springer, T. A., *Nature 346*:425-434 (1990)). Regulation of the neutrophil adhesive state is critical for leukocyte function. One major neutrophil adhesion molecule, the Mac-1 b2 integrin (CD11b/CD18), is both quantitatively upregulated (Bainton, D. F. *et al*., *J. Exp. Med*. *166*:1641-1653 (1987), Todd, R. F. III, *et al*., *J*. *Clin. Invest. 74:*1280-1290 (1984)) and assumes an active conformation upon neutrophil exposure to chemotactic factors (Buyon, J. P. *et al*., *J. Immunol*. *140:*3156-3160 (1988)). In contrast, another major neutrophil adhesion molecule, L-selectin, is rapidly down regulated upon neutrophil activation (Berg, M. *et al*., *Blood 76*:2381-2388 (1990); Griffin, J. D. *et al*., *J. Immunol. 145:*576-584 (1990); Jutila, M. A. *et al*., *J. Immunol. 143:*3318-3324 (1989); Kishimoto, T. K. *et al*., *Science 245:*1238-1241 (1989)). However antibodies against either Mac-1 or L-selectin are effective in blocking neutrophil recruitment to sites of inflammation *in vivo* (Jutila, M. A. *et al*., *J*. *Immunol*. *143:*3318-3324 (1989); Lewinsohn, D. M. *et al*., *J*. *Immunol*. *138:*4313-4321 (1987); Rosen, H. *et al*., J. Exp. Med. 166:1685-1701 (1987); Watson, S. R. *et al*., *Nature 349:*164-167 (1991)) and neutrophil adhesion to stimulated endothelial cells *in vitro* (Hallmann, R. *et al*., *Biochem. Biophys. Res. Commun. 174:*236-243 (1991); Harlan, J. M. *et al*., *Blood 66:*167-178 (1985); Kishimoto, T. K. *et al*., *Blood 78:*805-811 (1991); Smith, C. W. *et al*., *J. Clin. Invest. 87:*609-618 (1991); Smith, C. W. *et al*., *J. Clin. Invest. 83:*2008-2017 (1989); Spertini, O. *et al*., *J. Immunol*. *147:*2565-2573 (1991); Zimmerman, G. A. *et al*., *J. Clin. Invest.81:*531-537 (1988)). The observation that L-selectin and Mac-1 are inversely regulated upon activation provided the theoretical basis for a model of neutrophil-endothelial cell interaction (Kishimoto, T. K. *et al*., *Science 245:*1238-1241 (1989)), where L-selectin is involved in the early adhesion events, while Mac-1 is required for adhesion strengthening and transendothelial migration. The requirement for L-selectin in neutrophil rolling, the earliest observed interaction between circulating neutrophils and inflamed endothelium, has been demonstrated *in vivo* by intravital microscopy (Ley, K. *et al*., *Blood 77:*2553-2555 (1991); Von Andrian, U. H. *et al*., *Proc. Natl. Acad. Sci. USA 88:*7538-7542 (1991)) and *in vitro* by defined flow chamber studies (Abbassi, O. *et al*., *J*. *Immunol*. *147:*2107-2115 (1991); Smith, C. W. *et al.*, *J. Clin. Invest. 87:*609-618 (1991)). The transition from selectin-dependency to b2 integrin-dependency is triggered by chemotactic factors. The precise regulation and the rapid kinetics of L-selectin down regulation is suggestive of a role in the progression of neutrophil emigration.

Normal lymphocyte traffic to peripheral lymph nodes is also mediated by L-selectin. L-selectin is down regulated on activated lymphocytes, although with a slower time-course than that of neutrophils (Hamann, A. *et al*., J. Immunol. 140:737-743 (1988); Jung, T. M. *et al., J. Immunol*. *144:*3130-3136 (1990); Jung, T. M. *et al*., *J*. *Immunol*. *141:*4110-4117 (1988); Jutila, M. A. *et al*., *Blood 76:*178-183 (1990); Spertini, O. *et al*., *Leukemia 5:*300-308 (1991)). Previous studies have shown that a large fragment of L-selectin, corresponding to the expected size of the extracellular portion of L-selectin, can be recovered from the supernatant of activated neutrophils (Berg, M. *et al*., *Blood 76:*2381-2388 (1990); Kishimoto, T. K. *et al*., *Science 245:*1238-1241 (1989)) and lymphoid cells (Jung, T. M. *et al*., *J. Immunol. 144:*3130-3136 (1990)). A soluble form of L-selectin is found at high concentrations (1.5 - 2.0 mg/ml) in normal human serum (Schleiffenbaum, B. *et al*., *J. Cell Biol. 119:*229-238 (1992)) and also found in culture supernatants of PHA lymphoblasts (Spertini, O. *et al., J. Immunol*. *Methods 156:*115-123 (1992)) and L-selectin-transfected COS cells (Lasky, L. A. *et al*., *Cell 56:*1045-1055 (1989)). Based on the rapid kinetics of L-selectin down regulation, it has been postulated that L-selectin is cleaved by an activated membrane protease (Berg, M. *et al*., *Blood 76:*2381-2388 (1990); Jung, T. M. *et al*., J. *Immunol*. *144:*3130-3136 (1990); Kishimoto, T. K. *et al*., *Science 245:*1238-1241 (1989)). Low levels of exogenous chymotrypsin can selectively remove L-selectin from the neutrophil surface (Jutila, M. A. *et al*., *Cell*. *Immunol*. *132:*201-214 (1991)). However, chymotrypsin inhibitors, such as TPCK and PMSF, have no effect on L-selectin shedding in response to neutrophil activation (Campanero, M. R. *et al*., *Eur. J. Immunol*. *21:*3045-3048 (1991). and unpublished observations). Recently Jutila and colleagues (Palecanda, A. *et al*., *Eur. J. Immunol*. *22:*1279-1286 (1992)) have reported that crosslinking L-selectin, even at reduced temperatures, can cause down regulation of L-selectin in the absence of overt neutrophil activation. Camerini et al. (Camerini, D. *et al*., *Nature 342:*78-80 (1989)) isolated a cDNA encoding a PI-linked form of L-selectin and suggested that cleavage of the PI anchor may be a mechanism to down modulate L-selectin expression. However the physiological relevance of the PI-linked form has been questioned (Berg, M. *et al*., *Blood 76:*2381-2388 (1990)). Thus the precise mechanism of L-selectin down regulation remains unclear.

Recently the concept of membrane proximal cleavage of transmembrane proteins has emerged as a specific and inducible mechanism to rapidly modulate cell surface expression of diverse proteins (Ehlers, M. R. W. *et al*., *Biochemistry 30:*10065-10074 (1991)). Many growth factors and cytokines, such as TGF-a (48), CSF-1 (Stein, J. *et al*. *Oncogene 6:*601-605 (1991)), and Kit ligand (Huang, E. J. *et al., Mol. Biol. Cell 3:*349-362 (1992), have a transmembrane bound form which can be specifically released from the cell surface by a proteolytic mechanism upon cell activation. Similarly cell surface receptors and ectoenzymes, such as the TNF receptor (Kohno, T. *et at*., *Proc. Natl. Acad. Sci. USA 87:*8331-8335 (1990); Porteu, F. *et al*, *J*. *Exp. Med*. *172:*599-607 (1990)), folate receptor (Elwood, P. C. *et al*., *J*. *Biol. Chem. 266:*2346-2353 (1991)), b amyloid protein (Palmert, M. R. *et al*., *Proc. Natl. Acad. Sci. USA 86:*6338-6342 (1989); Weidemann, A. *et al*., *Cell 57:*115-126 (1989)), and angiotensin converting enzyme (ACE) (Ehlers, M. R. W. *et al., Proc. Natl. Acad. Sci. USA 88:*1009-1013 (1991); Wei, L. *et al*., *J. Biol. Chem. 266:*5540-5546 (1991)) can be cleaved to release a soluble form. It is possible that the mechanism for rapid down modulation of L-selectin is related to modulation of these other critical cell surface proteins.

### Summary of the Invention

The present invention relates to the discovery that during the activation and recruitment phase of L-selectin mediated neutrophil adhesion and migration, L-selectin is proximally cleaved from the surface of the neutrophil, thus generating a soluble form of L-selectin of approximately 70 kd. The 70 kd specie has been seen during, and is associated with, many types of inflammatory responses. In addition, the present invention discloses that a 6 Kd protein remains associated with the neutrophil after L-selectin cleavage. This previously unidentified 6 Kd species consists of the transmembrane and cytoplasmic domain of L-selectin (SEQ.. ID No. 2 and 3, respectively).

Based on these observations, the present invention provides methods of inhibiting cleavage of L-selectin comprising the step of contacting a cell expressing L-selectin with a L-selectin cleavage inhibitory agent, wherein said agent inhibits proteolytic cleavage within the amino acid sequence KLDKSFSMIKEGDYN (SEQ. ID No. 1).

The L-selectin cleavage inhibitors of the present invention can be used to reduce the severity of the various biological responses which are mediated by the stimulation or activation of L-selectin expressing cells. Many inflammatory reactions are due to reactions involving neutrophils which express L-selectin. The use of L-selectin cleavage inhibitors therefore provides a method of treating conditions which include, but are not limited to, inflammatory reactions and other conditions associated with the adhesion or migration (trafficking) of neutrophils, monocytes, lymphocytes, and other white blood cell types.

The present invention further provides methods for identifying inhibitors of L-selectin cleavage. For example, in initially evaluating potential inhibitors of L-selectin cleavage, cells expressing L-selectin are incubated with a prospective agent under conditions which would normally lead to L-selectin cleavage. In one example of such an assay, an L-selectin cleavage inhibitor can be identified by:
a) contacting a cell expressing L-selectin with an agent;
b) incubating the cell of step (a) under condition in which L-selectin would normally be cleaved; and
c) detecting whether the L-selectin is cleaved.

Alternatively, an isolated peptide containing the L-selectin cleavage site can be used to identify L-selectin cleavage inhibitors. For example, L-selectin cleavage inhibitors can be identified by;
a) contacting an agent with a protein containing the amino acid sequence KLDKSFSMIKEGDYN (SEQ. ID No. 1);
b) incubating the product of step (a) under condition which allows cleavage at a residue within SEQ. ID No. 1; and
c) determining whether said agent inhibits the cleavage of said SEQ. ID No. 1.

The present invention further provides methods for detecting L-selectin cleavage which are based on the observation that upon cleavage, a 6 Kd L-selectin species remains associated with the activated/stimulated cell. In detail, L-selectin cleavage can be detected by:
(a) incubating a cell, or an extract thereof, in the presence of an agent which is capable of selectively binding to the transmembrane domain of L-selectin; and
(b) determining whether said cell, or said cellular extract, binds to said agent.

Alternatively, L-selectin cleavage can be detected by:
(a) incubating a cell, or an extract thereof, in the presence of an agent which is capable of selectively binding to the cytoplasmic domain of L-selectin; and
(b) determining whether said cell, or cellular extract, binds to said agent.

### Brief Description of the Figures

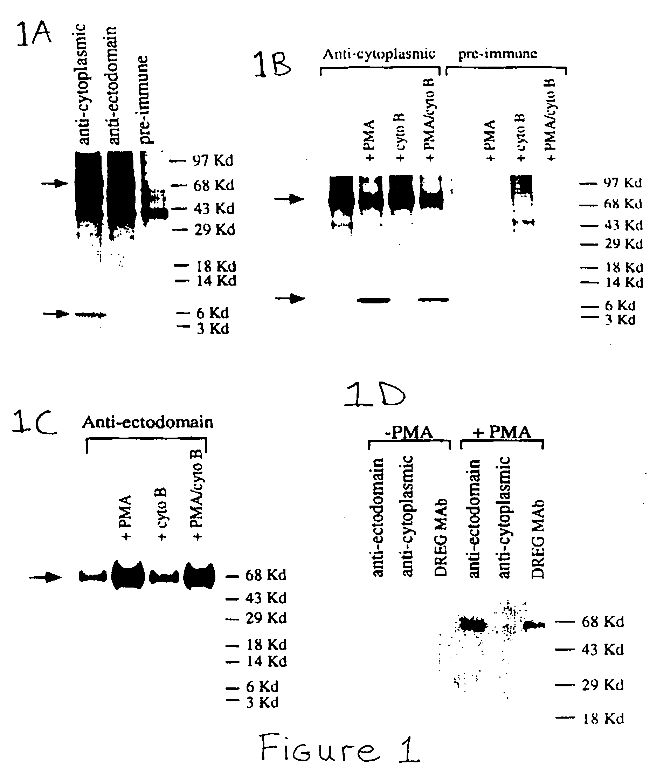

Figure 1 (Panels A-D). Identification of a 6 Kd species in metabolically labeled PHA lymphoblasts.

Peripheral blood lymphocytes were stimulated with PHA for five days. Lymphoblasts were pulse-labeled with [35S] -methionine for 15 min and chased with cold methionine for 30 min. Cells were treated with PMA (100 ng/nl) or cytochalasin B for 30 min, as indicated.

Panels A) and B) labeled cells were lysed in 1% Triton X-100 solution, and cell lysates were immunoprecipitated with antiserum directed against the ectodomain of L-selectin (JK923), the cytoplasmic domain of L-selectin (JK564) or with pre-immune serum, as indicated.

Panels C) and D). Cell free supernatants were immunoprecipitated with antisera against the ectodomain or the cytoplasmic domain of L-selectin or with the DREG-56 MAb, as indicated. Immunoprecipitates were subjected to SDS-PAGE on 10-20% gradient gels and visualized by autoradiography with fluorography.

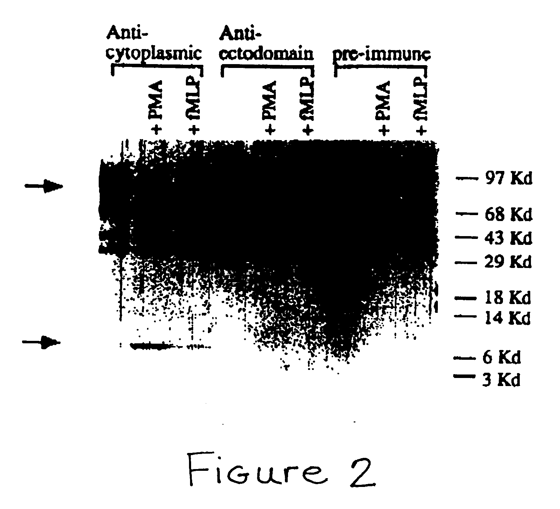

Figure 2. The 6 Kd species is associated with activated neutrophils.

Peripheral blood neutrophils were metabolically labeled with [35S]-methionine for five hours without chase, as described previously (Granelli-Piperno, A. *et al*., *J. Exp. Med. 149:*284 (1979); Loenen, W. A. M. *et al*., *Eur. J. Immunol. 22:*447-455 (1992)). Cells were treated with fMLP or PMA in the last 30 min of the labeling period, as indicated. Cell lysates were immunoprecipitated with antisera against the cytoplasmic domain, the ectodomain, or with preimmune serum, as indicated. Immunoprecipitates were analyzed as described in Figure 1. The mature (L) and precursor (L') forms of L-selectin are indicated.

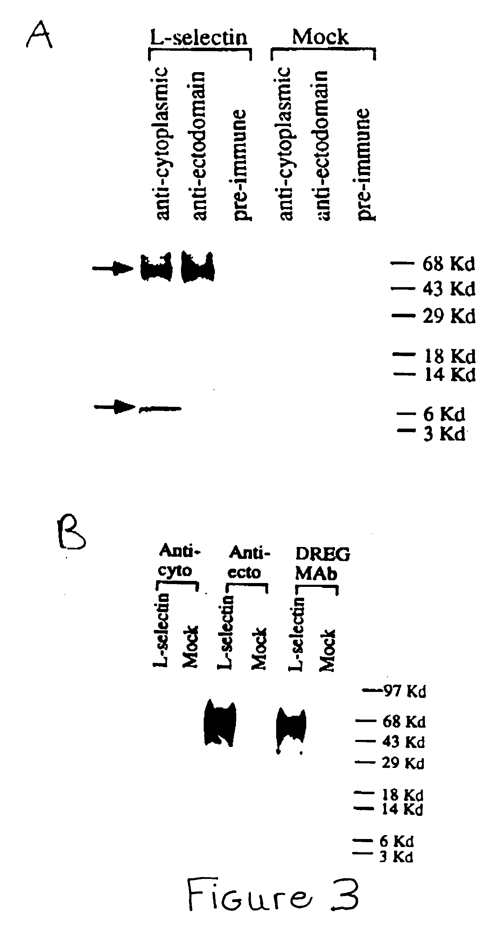

Figure 3 (Panels A-B). The 6 Kd species is a fragment of L-selectin.

COS cells were transiently transfected with a cDNA clone encoding the membrane form of L-selectin or were mock-transfected, as indicated. On day 3 post-transfection, cells were pulse-labeled with [35S]-methionine for 60 min and chased for 120 min.

Panel A). Cell lysates were immunoprecipitated with antisera directed against the cytoplasmic domain of L-selectin, the ectodomain of L-selectin, or with preimmune serum, as indicated.

Panel B). Cell-free supernatants of labeled COS cells were immunoprecipitated with polyclonal antiserum or the DREG-56 MAb, as indicated.

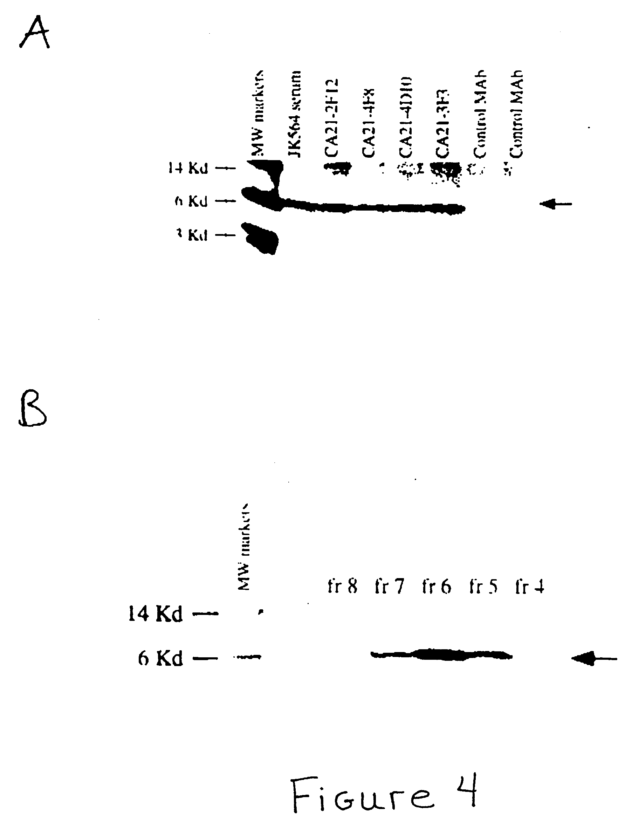

Figure 4 (Panels A-B). CA-21 MAb directed against the L-selectin cytoplasmic domain detects the 6 Kd species.

Panel A) COS cells were transfected with full-length L-selectin cDNA and labeled with [35S]-methionine, as described in Fig 3 legend. Cell lysates were immunoprecipitated with JK564 anti-cytoplasmic domain serum, the CA21 series of MAb (CA21-2E12, CA21-4F8, CA21-4D10, CA21-3F3), or irrelevant control MAb, as indicated. Immunoprecipitates were subjected to SDS-PAGE and autoradiography.

Panel B) Lysates of activated neutrophils were partially purified on a CA21-2E12 anti-L-selectin cytoplasmic domain MAb affinity column, subjected to SDS-PAGE and transferred to Immobilon PSQ membrane. The membrane was blocked in 1 % BSA and probed with CA21-2E12 MAb. Bands were visualized by addition of a second stage goat anti-mouse IgG-alkaline phosphatase-conjugate followed by development with BCIP and NBT. Prestained molecular weight standards were used to monitor transfer efficiency.

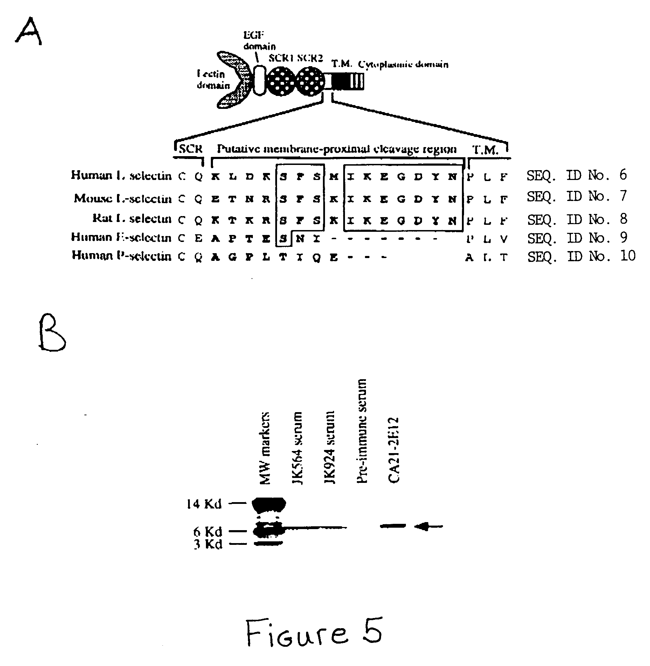

Figure 5 (Panels A-B). The 6 Kd species is an L-selectin transmembrane peptide (L-STMP).

Panel A). Sequence of the putative membrane-proximal cleavage region of human L-selectin, based on the size estimate of the 6 Kd species, aligned with the corresponding regions of mouse and rat L-selectin and human E-selectin and P-selectin. Conserved residues in this region are boxed, and the C-terminus of the last SCR domain and the N-terminus of the transmembrane domain are indicated.

Panel B). A rabbit antiserum (JK924) was raised against a synthetic peptide corresponding to the putative cleavage region as described in Materials and Methods. Lysates of L-selectin-transfected COS cells metabolically labeled with [35S]-methionine were immunoprecipitated with JK924 antisera against the putative cleavage region, JK564 anti- cytoplasmic domain serum, CA21 anti-cytoplasmic domain MAb, or preimmune serum, as indicated.

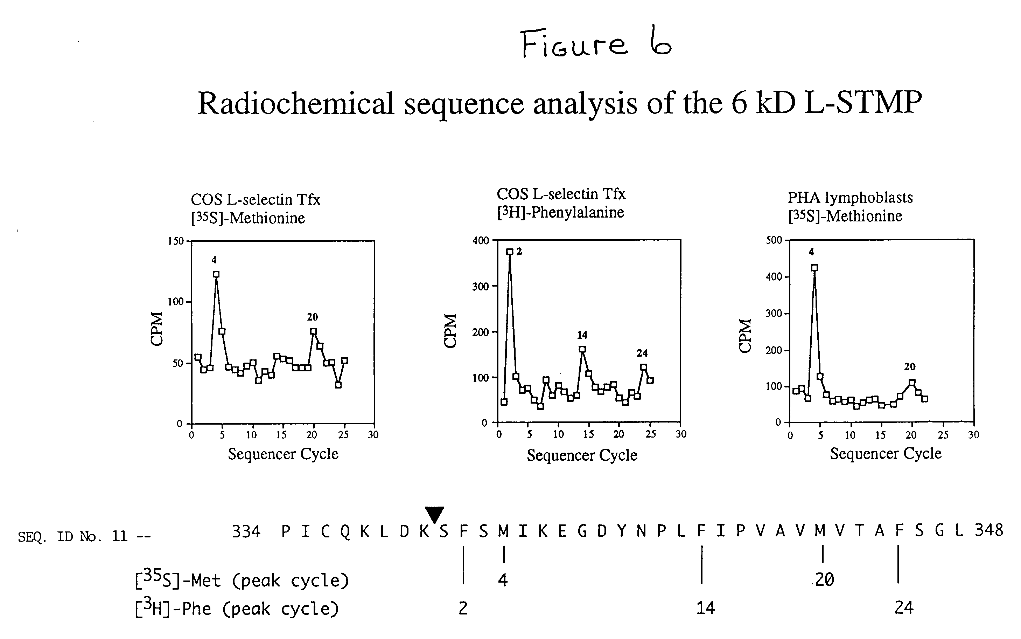

Figure 6. Radiochemical sequence analysis of the 6 Kd L-STMP.

Sequencer profile of radiolabeled amino acids release from cleaved 6 Kd L-STMP and the identification of the L-selectin cleavage site.

### Description of the Preferred Embodiments

The present invention is based on the observation that upon stimulation (activation), L-selectin molecules present on the surface of neutrophils undergo membrane proximal cleavage. As a results of the cleavage of L-selectin, a soluble 70 Kd species of L-selectin is liberated from the cell while a 6 Kd species of L-selectin remains associated with the stimulated cell. The present invention is further based on the identification of the amino acid sequence of the cleavage site of L-selectin.

Based on these observations, the present invention provides methods of inhibiting cleavage of L-selectin comprising the step of contacting a cell expressing L-selectin with a L-selectin cleavage inhibitory agent, wherein said agent inhibits proteolytic cleavage within the amino acid sequence KLDKSFSMIKEGDYN (SEQ. ID No. 1).

As used herein, inhibition of L-selectin cleavage is defined as a reduction in the rate or amount of L-selectin cleavage which occurs as a result of the presence of the cleavage inhibitor. Various methods can be used to determine the level of inhibition obtained. These include, but are not limited to assays which determine the presence of cleaved L-selectin (70 kd species or 6 kd species) and assays which determine the level of attachment or migration of the L-selectin expressing cells (cell adhesion/binding assays, see example 1).

As used herein, a cell is said to express L-selectin if the cell has been shown to possess L-selectin on the cell surface. Cells can be identified as expressing L-selectin using either immunological assays or though biological assays based on cell adhesion. A skilled artisan can readily employ known techniques in order to identify cells expressing L-selectin.

The L-selectin cleavage inhibitors of the present invention, and those used in the assay methods described below, include, but are not limited to, peptides, carbohydrates, vitamin derivatives, or other pharmaceutical agents. These agents can be selected and screened at random or rationally selected or designed using protein modeling techniques.

For random screening, agents such as peptides, carbohydrates, pharmaceutical agents and the like are selected at random and are assayed for their ability to inhibit L-selectin cleavage as outlined below. Alternatively, agents may be rationally selected or designed. As used herein, an agent is said to be "rationally selected or designed" when the agent is chosen based on the configuration of the L-selectin cleavage site or the structure of the protease involved in L-selectin cleavage. For example, one skilled in the art can readily adapt currently available procedures to generate antibodies, peptides, pharmaceutical agents and the like capable of binding to a specific peptide sequence such as the cleavage region of L-selectin, for example see Hurby et al., Application of Synthetic Peptides: Antisense Peptides", In *Synthetic Peptides, A User's Guide*, W.H. Freeman, NY, pp. 289-307 (1992), and Kaspczak *et al*., *Biochemistry 28*:9230-8 (1989) and Harlow, *Antibodies*, Cold Spring Harbor Press, NY (1990). Alternatively, inhibitors of the specific protease involved in L-selectin cleavage can be generated using procedures described elsewhere (Powers, J.C. *et al*., *Proteinase Inhibitors*, A.J. Barrett and G. Salvesen, Eds. chapters 3-6, pages 55-300, Elsevier, (1986)).

There are two main groups of L-selecting cleavage inhibitors. The first group (Group One Agents) comprises agents which interact with the L-selectin while the second group (Group Two Agents) comprises agents which interact with the L-selectin cleavage protease.

One type of L-selectin cleavage inhibitors which are especially preferred are antibodies. Group One antibodies comprise antibodies, or fragments thereof, which bind to the cleavage sequence of L-selectin (SEQ.. ID No. 1). The binding of such an antibody to L-selectin inhibits the cleavage of L-selectin. Group Two antibodies comprise antibodies, or fragments thereof, which bind to the L-selectin specific protease. The binding of such an antibody to the L-selectin protease, which cleaves within SEQ.. ID. No. 1, blocks the protease's ability to bind to or cleave the target sequence.

The antibodies utilized in the above methods can be monoclonal or polyclonal antibodies, as well fragments of these antibodies. In general, techniques for preparing monoclonal antibodies are well known in the art (Campbell, A.M., "*Monoclonal Antibody Technology: Laboratory Techniques in Biochemistry and Molecular Biology*," Elsevier Science Publishers, Amsterdam, The Netherlands (1984); Harlow; *Antibodies*, Cold Spring Harbor Press, NY (1989). For example, an antibody capable of binding to the L-selectin cleavage site can be generated by immunizing an animal with a polypeptide whose sequence comprises the L-selectin cleavage site (SEQ. ID No. 1 or 4, See Example 1).

Any animal (mouse, rabbit, etc.) which is known to produce antibodies can be utilized to produce antibodies with the desired specificity. Methods for immunization of these animals are well known in the art. Such methods include subcutaneous or intraperitoneal injection of the polypeptide. One skilled in the art will recognize that the amount of polypeptide used for immunization will vary based on the animal which is immunized, the antigenicity of the polypeptide selected, and the site of injection.

The polypeptide may be modified or administered in an adjuvant in order to increase the peptide antigenicity. Methods for increasing the antigenicity of a polypeptide are well known in the art. Such procedures include coupling the antigen with a heterologous protein (such as globulin or β-galactosidase) or through the inclusion of an adjuvant during immunization.

For generating monoclonal antibodies, spleen cells from the immunized animals are removed, fused with myeloma cells, such as SP2/0-Ag14 myeloma cells, and allowed to become monoclonal antibody producing hybridoma cells. Any one of a number of methods well known in the art can be used to identify the hybridoma cell which produces an antibody with the desired characteristics. These include screening the hybridomas with an ELISA assay, western blot analysis, or radioimmunoassay (Lutz *et al*., *Exp. Cell Res*. *175*:109-124 (1988); Kishimoto, T. K. *et al., Proc. Natl. Acad. Sci. USA 87:*2244-2248 (1990)). Hybridomas secreting the desired antibodies are cloned and the class and subclass of the secreted antibodies are determined using procedures known in the art (Campbell, A.M., *Monoclonal Antibody Technology: Laboratory Techniques in Biochemistry and Molecular Biology*, Elsevier Science Publishers, Amsterdam, The Netherlands (1984)). For polyclonal antibodies, antibody containing antisera is isolated from the immunized animal and is screened for the presence of antibodies with the desired specificity using one of the above-described procedures.

The present invention further includes other types of L-selectin cleavage inhibitors which can be generated using the amino acid sequence of the L-selectin cleavage site disclosed herein. One type of group one inhibitors are L-selectin cleavage inhibitors which are classified as antisense-peptide sequences. Antisense-peptide sequences are short peptide which are specifically designed to bind to a specific amino acid sequence. In general, such antisense peptide agents can be generated using methods known in the art, for example see Hurby et al., Application of Synthetic Peptides: Antisense Peptides", In *Synthetic Peptides, A User's Guide*, W.H. Freeman, NY, pp. 289-307 (1992), and Kaspczak *et al*., *Biochemistry 28*:9230-8 (1989).

Another type of Group Two L-selectin cleavage inhibitory agents comprise proteins which contain the actual L-selectin cleavage sequence. Such peptides inhibit L-selectin cleavage by interacting with, binding to, and diluting the activity of the L-selectin cleavage protease.

Another type of Group Two L-selectin cleavage inhibitors which are especially preferred are specific inhibitors of the protease involved in L-selectin cleavage. Although this protease has not been fully characterized, protease specific inhibitors can be generated based on the amino acid sequence of the L-selectin cleavage site disclosed herein. For example, using modern modeling techniques, a skilled artisan can design L-selectin cleavage protease inhibitors using SEQ.. ID. No. 1 (Powers, J.C. *et al*., *Proteinase Inhibitors*, A.J. Barrett and G. Salvesen, Eds. chapters 3-6, pages 55-300, Elsevier, (1986)).

The L-selectin cleavage inhibitors of the present invention can be used to reduce the severity of the various biological responses which are mediated by the stimulation or activation of L-selectin expressing cells. Many inflammatory reactions are due to reactions involving neutrophils which express L-selectin. The use of L-selectin cleavage inhibitors therefore provides a method of treating condition which include, but are not limited to, inflammatory reactions and other conditions associated with the adhesion or migration (trafficking) of neutrophils, monocytes, lymphocytes and other while blood cell types.

The L-selectin cleavage inhibitors of the present invention may be administered to a mammal intravenously, intramuscularly, subcutaneously, enterally, topically or parenterally in order to reduce the severity of biological responses which are associated with L-selectin cleavage (activation/stimulation of cells, such as neutrophils, which express L-selectin). When administering antibodies or peptides by injection, the administration may be by continuous injections, or by single or multiple injections.

The L-selectin cleavage inhibitors of the present invention are intended to be provided to a recipient mammal in a pharmaceutically acceptable form in an amount sufficient to be therapeutically effective. An amount is said to be therapeutically effective if the dosage, route of administration, etc. of the agent are sufficient to reduce the severity of an L-selectin mediated biological response. An L-selectin cleavage inhibitor is said to be pharmacologically acceptable if its administration can be tolerated by a recipient. The L-selectin cleavage inhibitors of the present invention can be formulated according to known methods of preparing pharmaceutically useful compositions, whereby these materials are combined with a pharmaceutically acceptable carrier vehicle. Suitable vehicles and their formulation, inclusive of other human proteins, e.g., human serum albumin, are well known and described in the art. In order to form a pharmaceutically acceptable composition which is suitable for effective administration, the compositions will contain an effective amount of one of the L-selectin cleavage inhibitors of the present invention together with a suitable amount of carrier. In addition to carriers, the antibody based inhibitors of the present invention may be supplied in humanized form.

Humanized antibodies may be produced, for example by replacing an immunogenic portion of an antibody with a corresponding, but non-immunogenic portion (i.e. chimeric antibodies) Cabilly, S. *et al*, European Patent Application 125,023; Better, M. *et al*., *Science 240*:1041-1043 (1988); Wood, C.R. *et al*., *Nature 314*:446-449 (1985)); Shaw *et al*., *J. Natl.Cancer Inst. 80*:1553-1559 (1988).

In providing an antibody based L-selectin cleavage inhibitor, the dosage of administered agent will vary depending upon such factors as the patient's age, weight, height, sex, general medical condition, previous medical history, etc. In general, it is desirable to provide the recipient with a dosage of the antibody which is in the range of from about 1 pg/kg to 10 mg/kg (body weight of patient), although a lower or higher dosage may be administered.

In providing a peptide based L-selectin cleavage inhibitor, the dosage of administered agent will vary depending upon such factors as the patient's age, weight, height, sex, general medical condition, previous medical history, etc. In general, it is desirable to provide the recipient with a dosage of the peptide which is in the range of from about 1 pg/kg to 10 mg/kg (body weight of patient), although a lower or higher dosage may be administered.

The L-selectin cleavage inhibitors of the present invention may be administered either alone or in combination with one or more additional immunosuppressive agents. The administration of such compound(s) may be for either a "prophylactic" or "therapeutic" purpose. When provided prophylactically, the immunosuppressive compound(s) are provided in advance of any L-selectin mediated response or symptom (for example, prior to, or shortly after the time of neutrophil trafficking but in advance of any physiological symptoms). The prophylactic administration of the compound(s) serves to prevent or reduce any subsequent L-selectin response (such as, for example, additional neutrophil trafficking). When provided therapeutically, the compound(s) is provided at (or shortly after) the onset of a symptom of actual L-selectin mediated response (such as, for example, the appearance of symptoms associated with neutrophil trafficking). The therapeutic administration of the compound(s) serves to reduce any actual L-selectin mediated response (such as, for example, reducing the damage associated with neutrophil trafficking). Therefore, the L-selectin cleavage inhibitors of the present invention may, be provided either prior to the onset of a L-selectin mediated response (so as to suppress an anticipated response) or after the initiation of the response.

Additional pharmaceutical methods may be employed to control the duration of action of the L-selectin cleavage inhibitor. Control release preparations may be achieved through the use of polymers to complex or absorb the agents of the present invention. The rate and duration of the controlled delivery may be regulated to a certain extent by selecting an appropriate macromolecule matrix, by varying the concentration of macromolecules incorporated, as well as the methods of incorporation. Another possible method to control the duration of action by controlled release preparations is to incorporate the agents of the present invention into particles of a polymeric material, such as polyesters, polyamino acids, hydrogels, poly(lactic acid) or ethylene vinyl acetate copolymers. Alternatively, instead of incorporating these agents into polymeric particles, it is possible to entrap these materials in microcapsules prepared, for example, by coacervation techniques or by interfacial polymerization, by gelatine or poly(methylmethacylate) microcapsulation, or in colloidal drug delivery systems, for example, liposomes, albumin microspheres, microemulsions, nanoparticles, and nanocapsules or in macroemulsions.

The invention further includes a pharmaceutical composition comprising the L-selectin cleavage inhibitors of the present invention alone, or in combination with at least one other immunosuppressive agent. Examples of suitable immunosuppressive agents include, but are not limited to, dexamethasone, azathioprine and cyclosporin A.

The sequence of the L-selectin cleavage site, as well as the sequence specific protease inhibitors of the present invention, can be used to identify, label, or purify the L-selectin protease. Specifically, the L-selectin cleavage site sequence can be used to purify the L-selectin protease using known methodologies. Specifically, the L-selectin cleavage site can be crosslinked to the protease or immobilized on a solid support and used as an affinity matrix to isolate L-selectin protease.

The present invention further provides methods for identifying inhibitors of L-selectin cleavage. For example, in initially evaluating potential inhibitors of L-selectin cleavage, cells expressing L-selectin are incubated with a prospective agent under conditions which would normally lead to L-selectin cleavage. In one example of such an assay, an L-selectin cleavage inhibitor can be identified by:
a) contacting a cell expressing L-selectin with an agent;
b) incubating the cell of step (a) under condition in which L-selectin would normally be cleaved; and
c) detecting whether the L-selectin is cleaved.

The cells used in the above assay can be cells which naturally express the L-selectin, or alternatively, can be cells which do not normally express L-selectin but which have been modified to express L-selectin. Procedures for introducing and expressing L-selectin within a cell are well known in the art (for example see, Sambrook; *Molecular Cloning*, Cold Spring Harbor Press, NY (1990). The cells used in the above method can be either intact cells, or extracts thereof (see below).

As described above, both immunological detection methods, (for example, assays which use antibodies to detect the cleaved L-selectin species), and functional assays (for example, assays which detect L-selectin modified attachment or migration), can be employed to detect whether the L-selectin is cleaved. Additional methods for detecting L-selectin cleavage are described in more detail below.

Alternatively, an isolated peptide containing the L-selectin cleavage site can be used to identify L-selectin cleavage inhibitors. For example, L-selectin cleavage inhibitors can be identified by;
a) contacting an agent with a protein containing the amino acid sequence KLDKSFSMIKEGDYN (SEQ. ID No. 1);
b) incubating the product of step (a) under condition which allow cleavage at a residue within SEQ. ID No. 1; and
c) determining whether said agent inhibits the cleavage of said SEQ. ID No. 1.

The present invention further provides methods for detecting L-selectin cleavage which are based on the observation that upon cleavage, a 6 Kd L-selectin species remains associated with the activated/stimulated cell. In detail, L-selectin cleavage can be detected by:
(a) incubating a cell, or an extract thereof, in the presence of an agent which is capable of selectively binding to the transmembrane domain of L-selectin; and
(b) determining whether said cell, or said cellular extract, binds to said agent.

Alternatively, L-selectin cleavage can be detected by:
(a) incubating a cell, or an extract thereof, in the presence of an agent which is capable of selectively binding to the cytoplasmic domain of L-selectin; and
(b) determining whether said cell, or cellular extract, binds to said agent.

The preferred agents used in the above described assay methods are antibodies which selectively bind the transmembrane domain or the cytoplasmic domain of L-selectin (SEQ. ID No. 2 and 3). Such antibodies, monoclonal as well as polyclonal, can be generated using known procedures. Such methods are described in more detail above and in Example 1.

The tissues, or cells used in the assays described above include, but are not limited to, cells, protein or membrane extracts of cells, or biological fluids such as blood, serum, plasma, or urine. The sample used in the above-described assays will vary based on the assay format, nature of the detection method and the tissues, cells or extracts used as the sample to be assayed. Methods for preparing protein extracts or membrane extracts of cells are well known in the art and can be readily be adapted in order to obtain a sample which is capable with the system utilized.

Conditions for incubating an antibody with a sample vary. Incubation conditions depend on the format employed in the assay, the detection methods employed, and the type and nature of the antibody used in the assay. One skilled in the art will recognize that any one of the commonly available immunological assay formats (such as radioimmunoassays, enzyme-linked immunosorbent assays, diffusion based Ouchterlony, or rocket immunofluorescent assays) can readily be adapted to employ the antibodies of the present invention. Examples of such assays can be found in Chard, T. "*An Introduction to Radioimmunoassay and Related Techniques*" Elsevier Science Publishers, Amsterdam, The Netherlands (1986); Bullock, G.R. *et al*., "*Techniques in Immunocytochemistry*," Academic Press, Orlando, FL Vol. 1 (1982), Vol. 2 (1983), Vol. 3 (1985); Tijssen, P., "*Practice and Theory of Enzyme Immunoassays: Laboratory Techniques in Biochemistry and Molecular Biology*," Elsevier Science Publishers, Amsterdam, The Netherlands (1985).

The materials and assays disclosed herein are ideally suited for the preparation of a kit. Specifically, the invention provides a kit compartmentalized to receive in close confinement, one or more containers which comprises: (a) a first container comprising one of the L-selectin binding agents of the present invention; and (b) one or more other containers comprising one or more of the following: a sample reservoir, wash reagents, and reagents capable of detecting the presence of bound binding agent from the first container.

In detail, a compartmentalized kit includes any kit in which reagents are contained in separate containers. Such containers include but are not limited to, small glass containers, plastic containers or strips of plastic or paper. Such containers allow one to efficiently transfer reagents from one compartment to another compartment such that the samples and reagents are not cross-contaminated and the agents or solutions of each container can be added in a quantitative fashion from one compartment to another. Such containers will include, but will not be limited to, a container which will accept the test sample, a container which contains antibodies used in the assay, containers which contain wash reagents (such as phosphate buffered saline, Tris-buffers, etc.), and containers which contain the reagents used to detect the bound antibody.

The types of detection reagents which can be used in the above described kits include, but are not limited to, labelled secondary probes, or in the alternative, if the primary probe or antibody is labelled, the enzymatic, or antibody binding reagents which are capable of reacting with the labelled probe. One skilled in the art will readily recognize that the antibody agents described above can be readily incorporated into one of the established kit formats which are well known in the art.

The detection of foci of L-selectin cleavage, through the use of labeled antibodies, can be used to diagnosis certain classes of L-selectin mediated responses. In one diagnostic embodiment, samples of tissue or blood are removed from a subject and are incubated in the presence of antibodies which are detectably labeled. In another embodiment, this technique is done in a non-invasive manner through the use of magnetic imaging, fluorography, etc.

Monoclonal antibodies capable of binding to either the cytoplasmic or transmembrane region of the 6 Kd L-selectin species may be employed as a means of imaging or visualizing the sites of L-selectin cleavage in a patient. In such a use, an anti-6 Kd monoclonal antibody is detectably labeled, through the use of radioisotopes, affinity labels (such as biotin, avidin, etc.), fluorescent labels, or paramagnetic atoms. Procedures for accomplishing such labeling are well known to the art. The labeled antibodies can then be used in diagnostic imaging. Clinical application of antibodies in diagnostic imaging are reviewed by Grossman, H.B., *Urol. Clin. North Amer. 13*:465-474 (1986)), Unger, E.C. *et al., Invest. Radiol. 20*:693-700 (1985)), and Khaw, B.A. *et al., Science 209*:295-297 (1980)).

Having now generally described the invention, the agents and methods of obtaining same will be more readily understood through reference to the following examples which are provided by way of illustration, and are not intended to be limiting of the present invention, unless specified.

### Example 1

### Materials and Methods

Polyclonal antisera. A polyclonal antiserum against the extracellular domain of L-selectin was generated by hyperimmunization of a rabbit with a purified preparation of a soluble, truncated form of L-selectin. An L-selectin cDNA lacking the coding sequence for the transmembrane and cytoplasmic domains was generated by PCR and subcloned into the CDM8 vector. COS cells were transfected with this construct by DEAE-dextran, as previously described (Camerini, D. *et al., Nature 342:*78-80 (1989)). Supernatants from transfected cells were harvested on days 3 - 14 after transfection and soluble L-selectin was purified on a DREG-200 anti-L-selectin MAb column (Kishimoto, T. K. *et al*., *Proc. Natl. Acad. Sci. USA 87:*2244-2248 (1990)).

A polyclonal antiserum against the cytoplasmic domain of L-selectin was generated by hyperimmunization of a rabbit with a BSA-conjugated 18 mer synthetic peptide corresponding to the entire cytoplasmic domain of L-selectin with an additional N-terminal cysteine residue (NH2-CRRLKKGKKSKRSMNDPY-COOH (SEQ. ID No. 4). The peptide was reduced and coupled via the sulfhydryl group of the N-terminal cysteine residue to maleimide activated BSA (Pierce, Rockford, Ill), according to the manufacturer's instructions. The N-terminal linkage provides an orientation similar to that expected of the cytoplasmic domain extending from the membrane bilayer. Serum titer was assayed for the ability to bind immobilized peptide.

A polyclonal antiserum against the membrane proximal region of the extracellular domain was generated by hyperimmunization of a rabbit with a KLH-conjugated 18 mer synthetic peptide corresponding to the membrane proximal region with an additional C-terminal cysteine residue (NH2-QKLDKSFSMIKEGDYNPC-COOH (SEQ. ID No. 5). The peptide was reduced and coupled to maleimide activated KLH as described above. The C-terminal linkage provides an orientation similar to that expected of the ectodomain extending from the membrane bilayer.

Monoclonal antibodies. The monoclonal antibody DREG-56 against the extracellular domain of L-selectin was prepared as previously described (Kishimoto, T. K. *et al*., *Proc. Natl. Acad. Sci. USA 87:*2244-2248 (1990)). Generation of the CA21 hybridoma cell lines producing MAb directed against the cytoplasmic domain of L-selectin is described in detail elsewhere (T.K. Kishimoto, F. Shirley, R. Rothlein, in preparation). Briefly, BALB/c mice were hyperimmunized with a synthetic peptide corresponding to the entire cytoplasmic domain of L-selectin, as described above. Spleen cells were fused with the SP2/0 myeloma fusion partner, as described previously (Kishimoto, T. K. *et al*., *Proc. Natl. Acad. Sci. USA 87:*2244-2248 (1990)). Hybridoma supernatants were screened for the ability to specifically recognize immobilized cytoplasmic domain peptide. Positive clones were further screened for the ability to immunoprecipitate L-selectin. CA21 (IgG1) MAb was purified by protein G affinity chromatography.

Metabolic labeling. COS cells were transiently transfected with cDNA encoding L-selectin (or mock transfected) by the DEAE-dextran method, as described previously (Camerini, D. *et al*., *Nature 342:*78-80 (1989)). Three days later cells were metabolically labeled with [35S]-methionine (New England Nuclear, Boston, MA). The COS monolayers were first preincubated in methionine-free RPMI-1640 containing 10% dialyzed FCS for 20 min at 37°C to deplete intracellular pools of methionine. Cells were pulse labeled with [35S]-methionine (0.5 - 1.0 mCi/100 mm dish) for various times, as indicated. The COS cells were then chased with complete RPMI 1640. Culture supernatants were harvested and centrifuged to remove any nonadherent cells or debris. Monolayers were lysed at 4°C in 1 ml of 1 % Triton X-100 in 10 mM Tris (pH 8)-saline-0.025 % azide (TSA) containing 1% ovalbumin, 1 mM aprotinin, and 1 mM freshly added PMSF (lysis buffer). Cells were removed from the plates simultaneously with lysis with a cell scraper, pipetted into microfuge tubes and incubated 30 min on ice. Cell nuclei were pelleted by centrifugation at 12000 X G.

Human mononuclear and polymorphonuclear (PMN) leukocytes were isolated from peripheral blood by ficoll-hypaque centrifugation and dextran sedimentation, as described previously (Smith, C. W. *et al., J. Clin. Invest*. *87:*609-618 (1991)). PHA-stimulated lymphoblasts were generated by incubating the mononuclear leukocytes with 2.5 mg/ml PHA (Sigma) in complete RPMI-1640 medium for 5 days at 37°C. The PHA lymphoblasts were harvested on day 5 and metabolically labeled with [35S]-methionine as described above. Freshly isolated human PMN were metabolically labeled as described (Granelli-Piperno, A. *et al*., *J. Exp. Med. 149:*284 (1979); McCormack, R. T. *et al., J. Immunol. 137:*1075-1082 (1986)). Briefly PMN were labeled at 2 X 10⁷ cells/ml for 5 hours at 37°C. PMA (100 ng/ml) or fMLP (10⁻⁶ M) was added in the final 30 min as indicated.

Immunoprecipitation and SDS-PAGE. Cell lysates were precleared with normal rabbit serum followed by protein A-agarose. One microliter of preimmune serum or specific serum was added to aliquots of the precleared lysates and cell-free supernatants. The samples were incubated at 4°C for 1-2 hours and then 15 ml of a protein A-agarose slurry was added to each sample. Samples were rotated end-over-end at 4°C for 30 min and then washed extensively. The specifically bound material was eluted by addition of SDS-PAGE sample buffer followed by incubation at 90°C for 10 min. Eluates were resolved on tricine-SDS polyacrylamide gradient (10-20%) gels (NOVEX, San Diego, CA.). Gels were subjected to fluorography (Enhance, NEN) and autoradiography.

Western blot analysis. An activated human granulocyte lysate was passed over a CA21 anti-cytoplasmic domain MAb column. The column was washed extensively and eluted with 50 mM glycine (pH 2) solution containing 1 % octyl thioglucopyranoside. Fractions were neutralized with Tris buffer and analyzed by dot blot analysis. Aliquots of partially purified material were subjected to SDS-PAGE on 10-20% polyacrylamide-SDS-tricine gels. Samples were transferred to Immobilon PSQ PVDF membrane (Millipore, Bedford, MA) in 10 mM CAPS buffer, pH11, containing 10% methanol. The membrane was blocked with 2% BSA and the transferred material was probed with CA21 anti-cytoplasmic tail MAb and detected with an alkaline phosphatase conjugated secondary antibody (Promega, Madison, WI) followed by BCIP and NBT, as described previously (Kishimoto, T. K. *et al., Proc*. *Natl. Acad. Sci. USA 87:*2244-2248 (1990)).

### Metabolic Labeling

COS cells were transfected with the cDNA encoding the human L-selectin molecule by the DEAE-dextran method. Four to six days later the cells were metabolically labeled with either [35S]-methionine (specific activity 10-130Ci/mmole). Cells were first preincubated in RPMI-1640 medium minus the amino acid that would be used in the biosynthetic label for 20 minutes at 37 C in order to deplete intracellular pools of the amino acid. Cells were pulse labeled with [35S]-methionine (0.5mCi/100mm dish, 10 total dishes) in RPMI-1640 devoid of methionine plus 10% dialyzed FCS for 1 hour at 37 C. The cells were then chased for 2 hours with RPMI-1640 containing 10% FCS (complete medium). COS cell monolayers were removed from the dishes with a cell scraper and simultaneously lysed in 1 % NP-40, 10mM Tris pH7.4, 150mM NaCl, 0.025% NaN3 plus 1mM each aprotinin and PMSF (lysis buffer). The cells were transferred to a conical tube and allowed to lyse on ice for 30 minutes. Cell nuclei were pelleted by centrifugation at 13,000 rpm. When labeling with [3H]-phenylalanine, the cells were treated as above with the following changes: The cells were pulse labeled for 5 hours in medium devoid of phenylalanine. The cells were chased and activated simultaneously by the addition of excess non-radioactive phenylalanine and PMA (100ng/ml) for 30 minutes. The cells were lysed by adding 2ml lysis buffer to a plate, scraping the cells, transferring the material to the next plate and repeating until all plates were done. The plates were then rinsed in an additional 2ml lysis buffer and pooled for a total of 4ml. The lysate was then treated as described above.

Human mononuclear cells were isolated from peripheral blood of healthy human donors by ficoll-lypaque centrifugation. Phyto-hemagglutinin (PHA)-stimulated lymphoblasts were generated by incubating the mononuclear leukocytes with 5ug/ml PHA in RPMI-1640 supplemented with 10% FCS (complete medium) for 6 days at 37 C, 5% CO2. The PHA-blasts (5x106-1x107/ml) were then metabolically labeled with either [35S]-methionine or [3H]-phenylalanine for 4-5 hours. The cells were then simultaneously chased with non-radioactive amino acid and activated with PMA (100ng/ml) for 30 minutes. Cell lysates were generated by incubating the cells with lysis buffer on ice and then pelleting the nuclei as described above.

### Immunoprecipitation, SDS-PAGE and Electrotransfer

Cell lysates were precleared with normal rabbit serum and protein A-agarose. The 6kD L-STMP was immunoprecipitated from the lysate with a rabbit polyclonal antisera (JK564) to the L-STMP in the following manner: 1 1:200 final dilution of JK564 was added to the lysate and allowed to react for 2 hours at 4 C on an end-over-end rotator. Protein A-agarose was added and incubated for 1-2 hours at 4 C on an end-over-end rotator. The agarose was washed extensively in Tris buffers and specifically-bound material was eluted by addition of Tricine SDS-PAGE sample buffer and heating the agarose at 95°C for 10 minutes. The eluted material was runon a Tricine SDS-polyacrylamide (10-20%) gradient gel (NOVEX, San Diego, CA). The material resolved on the gel was then electrotransferred to an Immobilon PSQ PVDF membrane (Millipore, Bedford, MA). The membrane was air-dried and exposed to Kodak X-OMAT AR film. The film was subsequently developed, aligned with the Immobilon membrane, and the area on the membrane corresponding to the point of migration of the 6kD L-STMP was excised and subjected to sequenator analysis.

### Results

Identification of a 6 Kd species on PHA lymphoblasts. We initially raised two antisera against L-selectin, one directed against purified soluble L-selectin which lacked the transmembrane and cytoplasmic domains (anti-ectodomain serum JK923) and another directed against a synthetic peptide corresponding to the entire cytoplasmic domain of L-selectin (anti-cytoplasmic domain serum JK564). The anti-ectodomain serum specifically stained intact, unactivated leukocytes, while the anti-cytoplasmic domain serum stained only permeabilized cells (data not shown). Both antisera immunoprecipitated the intact membrane-spanning form of L-selectin (Figure 1a and data not shown).

The ability of these antisera to detect processed fragments of L-selectin on activated leukocytes was examined. The primary sequence of L-selectin predicts six methionine residues, two of which are located in the cytoplasmic and transmembrane domains. A third methionine residue is located in the ectodomain, 10 amino acids proximal to the beginning of the transmembrane domain. Therefore we reasoned that both a transmembrane fragment of L-selectin as well as a soluble form of L-selectin should be detectable from activated cells metabolically labeled with [35S]-methionine. Lymphoblasts stimulated for 5 days with PHA actively synthesized L-selectin. The anti-ectodomain serum immunoprecipitated the intact 74 Kd L-selectin molecule from biosynthetically labeled cells (Figure 1a). Although PHA blasts are actively proliferating and constitutively produce a soluble form of L-selectin (Spertini, O. *et al., J. Immunol. Methods 156:*115-123 (1992)), addition of phorbol esters (PMA) provided a further activating signal which induced a significant increase in the down regulation of newly synthesized cell surface L-selectin. This is evidenced by reduced levels of intact L-selectin in immunoprecipitates from cell lysates (Figure 1b), increased levels of soluble L-selectin in immunoprecipitates from cell-free supernatants (Figure 1c), and decreased cell surface expression of L-selectin (data not shown). The anti-cytoplasmic domain serum also recognized the intact form of L-selectin (Figure 1a and b), but not the soluble form (Figure 1d). These results indicated that the classical transmembrane form of L-selectin is the major species found on these cells, and that the soluble form of L-selectin lacks the cytoplasmic tail. In addition, a faint band of 6 Kd could be reproducibly detected with the anti-cytoplasmic domain serum in cell lysates of day 5 PHA blasts (Figure 1a and b). Activation of lymphoblasts with PMA caused a marked decrease in the amount of detectable 74 Kd intact L-selectin and a concomitant marked increase in the amount of the 6 Kd species (Figure 1b). Addition of the microfilament disrupting agent cytochalasin B did not inhibit or enhance the down regulation of intact L-selectin or the appearance of the 6 Kd species (Figure 1b). These data suggested that the 74 Kd intact L-selectin species is cleaved upon activation with PMA to yield a 6 Kd cell bound fragment and a 68 Kd soluble fragment, and that this cleavage does not require cytoskeletal involvement.

A 6 Kd species is associated with PMA- and fMLP-activated neutrophils. Neutrophils also rapidly down regulate L-selectin in response to activation by chemotactic factors or phorbol esters. Although neutrophils are a terminally differentiated cell type with few ribosomes and limited endoplasmic reticulum, other groups have shown that neutrophil proteins can be metabolically labeled (Granelli-Piperno, A. *et al., J. Exp. Med. 149:*284 (1979); McCormack, R. T. *et al. J. Immunol. 137:*1075-1082 (1986)). Since L-selectin mRNA has been isolated from peripheral blood neutrophils (Ord, D. C. *et al., J. Biol. Chem. 265:* (1990)), we next examined if L-selectin biosynthesis could be detected. An intact form of L-selectin was immunoprecipitated with both the anti-ectodomain serum and the anti-cytoplasmic domain serum from neutrophils metabolically labeled with [35S]-methionine (Figure 2). As reported previously (Griffin, J. D. *et al., J*. *Immunol*. *145:*576-584 (1990); Lewinsohn, D. M. *et al*., *J*. *Immunol*. *138:*4313-4321 (1989)), the neutrophil form of L-selectin migrated slower than the lymphocyte form on SDS-PAGE, presumably due to additional post-translational modifications (Ord, D. C. *et al*., *J. Biol. Chem. 265:* (1990)). Activation of the neutrophils with either fMLP chemoattractant or phorbol esters resulted in greatly reduced amounts of intact L-selectin, with a corresponding increase in a 6 Kd species immunoprecipitated with the anti-cytoplasmic domain serum (Figure 2). This result was somewhat unexpected since prolonged culture of neutrophils necessary for metabolic labeling can induce neutrophil shape change and degranulation and might be expected to result in immediate L-selectin down regulation. However, these results suggested that down regulation of the newly synthesized L-selectin is greatly enhanced by a specific activating agent. As these cultures were not chased with cold methionine, a precursor form of L-selectin (L', Figure 2) was evident in these immunoprecipitations. The L-selectin precursor was not down regulated in response to PMA or fMLP (Figure 2), suggesting that only the mature cell surface form of L-selectin is susceptible to cleavage. The estimated size of the 6 Kd species is consistent with our previous observation that [125I]-surface labeled L-selectin released into the supernatant is approximately 4 Kd smaller than the membrane bound form (Kishimoto, T. K. *et al., Science 245:*1238-1241 (1989)).

The 6 Kd species is a specific product of L-selectin. To confirm that the 6 Kd species is directly related to L-selectin, we compared COS cells that were transfected with L-selectin cDNA versus those that were mock transfected. Lasky et al. (Lasky, L. A. *et al., Cell 56:*1045-1055 (1989)) have previously shown that L-selectin transfectants produce a soluble form of L-selectin. We confirmed this observation and extended it to demonstrate that the anti-cytoplasmic domain serum immunoprecipitated both the intact L-selectin and the 6 Kd species from L-selectin transfectants but not mock transfectants (Figure 3a). Moreover, the anti-cytoplasmic domain serum did not react with the soluble L-selectin from COS cell supernatants (Figure 3b). These results strongly indicate that the 6 Kd species is a specific product of L-selectin and that COS cells can constitutively process L-selectin in a manner similar to that of activated leukocytes.

Western blot analysis of the 6 Kd species. During the course of these experiments, a series of mouse MAb, CA21, directed against the cytoplasmic domain of L-selectin was developed. The CA21 MAb, like the JK564 polyclonal anti-cytoplasmic domain serum, immunoprecipitated the 6 Kd species from lysates of L-selectin-transfected COS cells (Figure 4a).

It was possible that the 6 Kd species co-precipitated with L-selectin and was not directly related to a fragment of L-selectin. This seemed unlikely given the correlation between the appearance of the 6 Kd species and the disappearance of the intact L-selectin on activated leukocytes. However, to formally exclude this possibility lysates of activated neutrophils were subjected to affinity chromatography followed by western blot analysis. The CA21 MAb reacted directly with both the intact form of L-selectin and the 6 Kd species by western blot analysis (Figure 4b). Control MAb showed no reactivity under these conditions (data not shown). These results indicated that the 6 Kd species is related to the cytoplasmic domain of L-selectin and also confirm that activated neutrophils can produce the 6 Kd species.

The 6 Kd L-selectin product is a transmembrane peptide. A 6 Kd C-terminal fragment of L-selectin would indicate a transmembrane peptide of approximately 50 to 55 amino acids. This would map the putative cleavage site to a short region of the ectodomain, between the C-terminus of the second short consensus repeat and the beginning of the transmembrane domain (Figure 5a). Interestingly this region is well conserved between mouse (Lasky, L. A. *et al., Cell 56:*1045-1055 (1989); Siegelman, M. H. *et al*. *Science 243:*1165-1172 (1989)), rat (Watanabe, T. *et al., Biochim. Biophys*. *Acta Gene Struct. Expression 1131:*321-324 (1992)) and human L-selectin (Bowen, B. R. *et al., J. Cell Biol. 109:*421-427 (1989); Camerini, D. *et al*., *Nature 342:*78-80 (1989); Siegelman, M. H. *et al., Proc. Natl. Acad. Sci*. *USA 86:*5562-5566 (1989); Tedder, T. F. *et al., J. Exp. Med. 170:*123-133 (1989)), but is not conserved between L-, E-, and P-selectin (Bevilacqua, M. P. *et al., Science 243:*1160-1165 (1989); Johnston, G. I. *et al., Cell 56:*1033-1044 (1989)) (Figure 5a). Although soluble forms of E-selectin and P-selectin have been described, neither protein is regulated in the same manner as L-selectin. To confirm that the 6 Kd species is a transmembrane product of L-selectin, we produced a third polyclonal serum (JK924) against a synthetic peptide (NH2-QKLDKSFSMIKEGDYNPC-COOH (SEQ.. ID No. 5)) corresponding to the sequence of this putative membrane-proximal cleavage domain with an additional C-terminal cysteine residue, as described in materials and methods. Both the JK924 anti-cleavage domain serum and the JK564 anti-cytoplasmic domain serum immunoprecipitated the 6 Kd species from cell lysates of L-selectin transfectants (Figure 5b). Thus the 6 Kd species reacted with two independent antisera, one directed against membrane-proximal extracellular determinants and the other directed against cytoplasmic domain determinants. These results indicated that the 6 Kd species is a transmembrane peptide fragment of L-selectin.

### Discussion

We have identified a 6 Kd L-selectin transmembrane peptide (L-STMP) on activated leukocytes and on L-selectin transfectants. The 6 Kd L-STMP reacted with monoclonal antibodies and polyclonal antiserum directed against the cytoplasmic domain of L-selectin and with an antiserum directed against a 17 amino acid region of the ectodomain adjacent to the transmembrane domain. Appearance of the 6 Kd L-STMP on activated leukocytes correlated with the disappearance of the intact membrane bound L-selectin and with the appearance of a soluble form of L-selectin in culture supernatants. Previous studies have estimated that the soluble form of L-selectin is 4 Kd (Kishimoto, T. K. *et al*., *Science 245:*1238-1241 (1989)) to 12 Kd (Jung, T. M. *et al., J*. *Immunol. 144:*3130-3136 (1990)) smaller than the intact membrane form. However, the precise nature of L-selectin processing has been difficult to determine because the large heavily glycosylated ectodomain migrates as a diffuse band by SDS-PAGE. In contrast the L-STMP migrated as a sharp band with an apparent molecular weight of 6.0 - 6.5 Kd. The estimated size of the L-STMP and soluble L-selectin is consistent with a single membrane proximal cleavage site, although we can not exclude the possibility of two or more closely spaced cleavage sites. The size of the 6 Kd form would indicate a putative cleavage site in a short region between the C-terminus of the second SCR and the beginning of the transmembrane domain. This estimate is also consistent with recognition of the 6 Kd species by an antiserum directed against a synthetic peptide corresponding to this membrane proximal extracellular region. Although the extracellular domains of L-, E-, and P-selectin are well conserved (Bevilacqua, M. P. *et al., Science 243:*1160-1165 (1989); Bowen, B. R. *et al., J. Cell Biol. 109:*421-427 (1989); Camerini, D. *et al., Nature 342:*78-80 (1989); Johnston, G. I. *et al., Cell 56:*1033-1044 (1989); Siegelman, M. H. *et al., Proc. Natl. Acad. Sci. USA 86:*5562-5566 (1989); Tedder, T. F. *et al., J. Exp. Med. 170:*123-133 (1989)), this short membrane proximal region is highly divergent. This is consistent with the observation that only L-selectin is rapidly down regulated in this manner. Taken together these results strongly support the model that L-selectin is cleaved at a membrane proximal site.

Down regulation of L-selectin on leukocytes can be triggered by specific cell activating agents (Berg, M. *et al., Blood 76:*2381-2388 (1990); Griffin, J. D. *et al., J. Immunol. 145:*576-584 (1990); Hamann, A. *et al., J*. *Immunol. 140:*737-743 (1988); Jung, T. M. et al., *J. Immunol. 144:*3130-3136 (1990); Jung, T. M. *et al., J. Immunol. 141:*4110-4117 (1988); Jutila, M.A. *et al*., *Blood 76:*178-183 (1990); Jutila, M. A. *et al., J. Immunol. 143:*3318-3324 (1989); Kishimoto, T. K. *et al., Science 245:*1238-1241 (1989); Spertini, O. *et al., Leukemia 5:*300-308 (1991)). These studies indicate that either a protease is mobilized or activated or that L-selectin undergoes a conformational change and becomes accessible to a constitutively active protease. Newly synthesized L-selectin on PHA lymphoblasts and cultured neutrophils still required an activating agent, such as PMA, for efficient L-selectin down regulation, even though these cells have already been stimulated to some degree. Jung and Dailey (Jung, T. M. *et al., J. Immunol. 144:*3130-3136 (1990)) reported that L-selectin down modulation in response to PMA activation is inhibited by staurosporine, an inhibitor of protein kinase C (PKC). However this is not entirely unexpected since PMA directly activates PKC. It is possible that L-selectin down regulation may also occur via PKC-independent pathways in response to chemotactic factors, as has been shown in the case of neutrophil aggregation in response to fMLP versus PMA (Merrill, J. T. *et al., J. Immunol. 145:*2608-2615 (1990)).

Other leukocyte membrane proteins, including CD14 (Bazil, V. *et al*., *J. Immunol. 147:*1567-1574 (1991)), CD16 (Harrison, D. *et al., J. Immunol*. *147:*3459-3465 (1991)), CD 27 (Loenen, W. A. M. *et al., Eur. J. Immunol*. *22:*447-455 (1992)), CD43 (Bazil, V. *et al., Proc. Natl. Acad. Sci. USA 90:*3792-3796 (1993); Campanero, M. R. *et al., Eur. J. Immunol*. *21:*3045-3048 (1991); Rieu, P. *et al., Eur. J*. *Immunol*. *22:*3021-3026 (1992)), CD 44 (Bazil, V. *et al., J. Immunol. 149:*747-753 (1992); Campanero, M. R. *et al*., *Eur. J. Immunol*. *21:*3045-3048 (1991)), and TNF receptor (Kohno, T. *et al*., *Proc. Natl. Acad. Sci. USA 87:*8331-8335 (1990); Porteu, F. *et al., J. Exp*. *Med. 172:*599-607 (1990)), have been reported to be down modulated upon leukocyte activation. However the rapid kinetics of L-selectin shedding are unusual. Soluble forms of CD27 (Loenen, W. A. M. *et al., Eur. J. Immunol. 22:*447-455 (1992)), FCgRII (Sarmay, G. *et al, Eur. J. Immunol. 21:*541-549 (1991)), and ICAM-1 (Rothlein, R. *et al., J. Immunol. 147:*3788-3793 (1991)) have been reported in the supernatant of stimulated lymphocytes and monocytes over a period of hours to days, although the mechanism that generate these soluble forms is unknown. CD43 and CD44 on neutrophils is down regulated over a period of 15 min to 60 min in response to PMA (Rieu, P. *et al., Eur. J. Immunol. 22:*3021-3026 (1992)), but this is in contrast to the cleavage of L-selectin within several minutes on the same cells. Moreover physiological stimuli, such as TNF and fMLP, are poor modulators of CD43 and CD44 cleavage, but cause efficient down regulation of L-selectin (Bazil, V. *et al., Proc. Natl. Acad. Sci. USA 90:*3792-3796 (1993)). CD16 (FcgRIII) is expressed as both a PI-linked form (CD16-I) on neutrophils and a transmembrane bound form (CD16-II) on NK cells. Both forms are released upon cell activation; however, release of CD16-II, but not CD16-I, appears to involve a proteolytic mechanism (Harrison, D. *et al., J. Immunol*. *147:*3459-3465 (1991)). CD14 is also a PI-linked protein, although release of CD14 appears to involve a proteolytic mechanism (Bazil, V. *et al., J*. *Immunol. 147:*1567-1574 (1991)). A cDNA encoding a PI-linked form of L-selectin has been reported (Camerini, D. *et al., Nature 342:*78-80 (1989)); however, L-selectin expression is not deficient in patients lacking PI-linked proteins (Berg, M. *et al., Blood 76:*2381-2388 (1990); Ord, D. C. *et al., J*. *Biol. Chem. 265:* (1990)). Common serine protease inhibitors, such as PMSF and aprotinin, have been shown to inhibit release of CD14 and CD44 (Bazil, V. *et al., J. Immunol. 147:*1567-1574 (1991); Campanero, M. R. *et al., Eur*. *J. Immunol. 21:*3045-3048 (1991)), while a metalloprotease inhibitor, 1,10 phenanthroline, inhibits release of CD16-II (Harrison, D. *et al., J. Immunol*. *147:*3459-3465 (1991)). Interestingly, down regulation of CD43 appears to be sensitive to both serine and metalloprotease inhibitors (Bazil, V. *et al*., *Proc. Natl. Acad. Sci. USA 90:*3792-3796 (1993); Campanero, M. R. *et al*., *Eur. J. Immunol. 21:*3045-3048 (1991); Rieu, P. *et al., Eur. J. Immunol*. *22:*3021-3026 (1992)) In contrast L-selectin down regulation has been reported to be resistant to the serine protease inhibitors, aprotinin or PMSF (Campanero, M. R. *et al., Eur. J. Immunol. 21:*3045-3048 (1991)). Moreover, we have found that L-selectin down regulation appears to be insensitive to a battery of inhibitors of serine proteases (PMSF, APMSF, aprotinin, antipain, leupeptin, TLCK, TPCK), metalloproteases (EDTA, phosporamidon, leupeptin, antipain, bestatin), aspartic proteases (pepstatin), and thiol proteases (E64) (unpublished observations). These negative results suggest either a novel protease or a protease that is activated or mobilized so rapidly upon cell activation that these inhibitors have no significant effect on L-selectin down regulation. These results suggest that the mechanism to down regulate L-selectin is distinct from the mechanism involved in down regulation of these other leukocyte cell surface proteins. Only a neutrophil TNF receptor has been reported to be down regulated with similar kinetics as that of L-selectin and to be insensitive to a battery of serine protease inhibitors (Porteu, F. *et al., J. Exp. Med. 172:*599-607 (1990)). Recently Jutila and colleagues (Palecanda, A. *et al., J. Immunol. 150:*304A.(Abstr.) (1993)) provided evidence that DFP, a potent serine protease inhibitor, can inhibit L-selectin release from activated leukocytes and L-selectin-crosslinked leukocytes. These studies taken together with our findings on the L-STMP product strongly support a proteolytic mechanism for rapid down regulation of L-selectin.

Membrane proximal cleavage of transmembrane domains is not limited to leukocytes, but rather appears to be involved in regulating cell surface expression of proteins on many cell types (Ehlers, M. R. W. *et al*., *Biochemistry 30:*10065-10074 (1991)). Proteins as diverse as the b-amyloid protein (Palmert, M. R. *et al., Proc. Natl. Acad. Sci. USA 86:*6338-6342 (1989); Weidemann, A. *et al., Cell 57:*115-126 (1989)) associated with Alzheimers disease, the angiotensin converting enzyme (Wei, L. *et al., J*. *Biol. Chem. 266:*5540-5546 (1991)), the folate receptor (Elwood, P. C. *et al*., *J. Biol. Chem. 266:*2346-2353 (1991)), growth factor and cytokine receptors, such as TNF receptor (Kohno, T. *et al., Proc. Natl. Acad. Sci. USA 87:*8331-8335 (1990); Porteu, F. *et al., J. Exp. Med. 172:*599-607 (1990)) and CSF-1 receptor (Downing, J. R. *et al., Mol. Cell. Biol*. *9989:*2890-2896 (1989)), as well as growth factors and cytokines themselves, such as TNF (Kriegler, M. *et al., Cell 53:*45-53 (1988)), TGF-a (Pandiella, A. *et al., J. Biol. Chem*. *267:*24028-24033 (1992)), CSF-1 (Stein, J. *et al. Oncogene 6:*601-605 (1991)), and Kit ligand (Huang, E. J. *et al., Mol. Biol. Cell 3:*349-362 (1992)), have transmembrane forms which are cleaved to release soluble forms. Down modulation of many of these proteins is greatly accelerated upon cell activation with PMA (Ehlers, M. R. W. *et al., Biochemistry 30:*10065-10074 (1991); Pandiella, A. *et al., Proc. Natl. Acad. Sci. USA 88:*1726-1730 (1991)). In many cases both the soluble and the transmembrane forms of these proteins have been shown to be biologically active. Membrane forms of cytokines may have directed, local effects, while the soluble form may have more systemic effects (Kriegler, M. *et al., Cell 53:*45-53 (1988); Pandiella, A. *et al., Proc. Natl. Acad. Sci. USA 88:*1726-1730 (1991)). Soluble L-selectin can inhibit L-selectin-dependent adhesion in vitro (Schleiffenbaum, B. *et al., J. Cell Biol. 119:*229-238 (1992)), although its *in vivo* physiological role is unknown. It is also unknown whether membrane proximal cleavage of any of these receptors results in transmission of a signal through the transmembrane fragment. Antisera against the cytoplasmic domain and the membrane-proximal extracellular region of L-selectin may allow us to address this question.

It is not clear whether these transmembrane proteins are cleaved by one common membrane proximal protease activity or multiple related activities. It is unusual that COS cells, which do not normally express L-selectin, can process L-selectin into fragments which are indistinguishable from those found with activated leukocytes. These results suggest that this protease activity may be ubiquitous or possibly intrinsic to L-selectin. There is precedent for autoproteolytic activity in proteins, although these proteins typically have conserved consensus sequences of one of the major protease families. L-selectin lacks any obvious homology to known proteases, although we can not exclude the possibility of a novel protease family. Letellier et al (Letellier, M. *et al., J. Exp. Med. 172:*693-700 (1990)) have provided evidence that the IgE Fc receptor, which also has a C-type lectin domain, may have autoproteolytic activity. Another possibility is that this or a related protease is expressed ubiquitously on a variety of cell types (including COS cells) and involved in the regulation of other cell surface molecules. Recently it has become apparent that a number of cleavable transmembrane proteins exhibit a similar pattern of site-specific cleavage in transfected COS or CHO cells (Bosenberg, M. W. *et al., Cell 71:*1157-1165 (1992); Kohno, T. *et al., Proc. Natl. Acad*. *Sci. USA 87:*8331-8335 (1990); Wei, L. *et al., J. Biol. Chem. 266:*5540-5546 (1991)), suggesting the possibility of a common proteolytic pathway. However differential susceptibility of these proteins to protease inhibitors indicate at least three distinct activities: one susceptible to serine protease inhibitors (Bazil, V. *et al., J. Immunol. 147:*1567-1574 (1991); Campanero, M. R. *et al., Eur. J. Immunol. 21:*3045-3048 (1991); Pandiella, A. *et al., J*. *Biol. Chem. 267:*24028-24033 (1992)), another susceptible to metalloprotease inhibitors (Harrison, D. *et al., J. Immunol. 147:*3459-3465 (1991)), and a third that is resistant to most common protease inhibitors (Letellier, M. *et al*., *Mol. Immunol. 26:*1105-1112 (1989); Porteu, F. *et al., J. Exp. Med. 172:*599-607 (1990)). Even these activities can be further subdivided since down regulation of some proteins are inhibited by some serine protease inhibitors but not others (Pandiella, A. *et al., J. Biol. Chem. 267:*24028-24033 (1992)). Moreover, the cleavage sites for some of these proteins have been determined, but have no significant regions of homology to each other or to the putative cleavage site of L-selectin. The cleavage of TGF-a and Kit ligand has been mapped to a region rich in small apolar residues, such as alanine and valine (Pandiella, A. *et al., J. Biol. Chem. 267:*24028-24033 (1992)), while the putative cleavage region of L-selectin is strikingly devoid of these residues. Positional effects or a protease docking site may also be important. In an elegant study, Bosenberg et al. (Bosenberg, M. W. *et al., Cell 71:*1157-1165 (1992)) have demonstrated that cleavage of the extracellular domain of membrane TGF-a is specified by the C-terminal valine residue of the cytoplasmic tail. These data indicate that the C-terminal end of the TGF-a cytoplasmic tail is a critical recognition site for a regulatory protein or a transmembrane protease.

The physiological requirement for rapid L-selectin down regulation remains unclear. It was originally postulated that L-selectin down regulation may be necessary for neutrophils to progress from initial contact to transendothelial migration (Kishimoto, T. K. *et al., Science 245:*1238-1241 (1989)). Alternatively L-selectin down regulation may serve as a protective mechanism to prevent neutrophils activated in circulation from entering an inappropriate tissue site. More recently, Palecanda et al. (Palecanda, A. *et al., Eur. J. Immunol. 22:*1279-1286 (1992)) showed that crosslinking of L-selectin causes its down regulation, and proposed that shedding of L-selectin may be a continuous process during neutrophil rolling and normal leukocyte trafficking. The continuous release of L-selectin during leukocyte trafficking and neutrophil rolling in response to subclinical stimuli may account in part for the high levels of circulating L-selectin in normal serum (Schleiffenbaum, B. *et al., J. Cell Biol. 119:*229-238 (1992)). The finding that cultured neutrophils actively synthesized L-selectin suggests the possibility that at least some of the neutrophil L-selectin can be replenished by de novo synthesis. It will be of interest to determine the biological consequences of interfering with this precisely regulated processing event. The anti-cytoplasmic domain serum and site-directed mutagenesis will provide a valuable tools to identify the precise cleavage site for L-selectin in order to further characterize the protease involved in modulation of this and perhaps other membrane proteins.

### Identification of the Cleavage Site

To determine the actual cleavage site, the 6kD species was purified for N-terminal sequence analysis. Since the deduced amino acid sequence of L-selectin is known, we performed radiochemical sequence analysis of the 6kD species from L-selectin-transfected COS cells biosynthetically labeled with [³⁵S]-methionine. The cell lysate was immunoprecipitated with the anti-cytoplasmic domain serum, subjected to SDS-PAGE, and transferred to PVDF membrane. The 6kD band was visualized by autoradiography, excised and subjected to a gas phase protein sequenator. Radioactive peaks were observed at cycles 4 and 20, indicating methionine residues at these positions. A spacing of 16 amino acids between the two methionine residues is found only between Met³²⁵ and Met³⁴¹ of L-selectin, suggesting that L-selectin was cleaved between Lys³²¹ and Ser³²². No radioactivity was observed with mock-transfected COS cells (data not shown). To confirm this predicted cleavage site, we performed radiochemical sequence analysis with COS transfectants biosynthetically labeled with another radiolabeled amino acid, [³H]-phenylalanine. Radioactive peaks were observed at cycles 2, 14, and 24, indicating phenylalanine residues at these sites. The spacing between the observed peaks was consistent only with the spacing between Phe³²³, Phe³³⁵, and Phe³⁴⁵ of L-selectin, again indicating a cleavage site between Lys³²¹ and Ser³²². Cleavage at this site would produce a transmembrane peptide with a predicted molecular mass of 5823 daltons, which is in excellent agreement with the observed Mr=6 kD by SDS-PAGE. To confirm that this is a natural cleavage site, and not an artifact of the COS system, we biosynthetically labeled PHA lymphoblasts with [³⁵S]-methionine and [³H]-phenylalanine. Again radioactive peaks were observed at cycles 4 and 20 with [³⁵S] methionine (Figure 6) and prominent peaks were observed at cycles 2 and 14 with [³H]-phenylalanine (data not shown). These results confirm that L-selectin is cleaved between Lys³²¹ and Ser³²² and indicate that COS cells process L-selectin in a manner indistinguishable from PHA lymphoblasts (Figure 6).

## Claims

1. A method of inhibiting cleavage of L-selectin comprising the step of contacting a cell expressing L-selectin with a L-selectin cleavage inhibitory agent, wherein said L-selectin cleavage inhibitory agent inhibits proteolytic cleavage within the amino acid sequence KLDKSFSMIKEGDYN (SEQ. ID No. 1).

2. The method of claim 1, wherein said L-selectin cleavage inhibitory agent is an antibody which is capable of binding to an epitope within SEQ. ID No. 1.

3. The method of claim 1, wherein said L-selectin cleavage inhibitory agent is a peptide which is capable of binding to SEQ. ID No. 1.

4. An isolated L-selectin cleavage inhibitory agent, wherein said agent inhibits proteolytic cleavage within the amino acid sequence KLDKSFSMIKEGDYN (SEQ. ID No. 1).

5. The L-selectin cleavage inhibitory agent of claim 4, wherein said L-selectin cleavage inhibitory agent is an antibody which is capable of binding to an epitope within SEQ. ID No. 1.

6. The L-selectin cleavage inhibitory agent of claim 4, wherein said L-selectin cleavage inhibitory agent is a peptide which is capable of binding to SEQ. ID No. 1.

7. A method for detecting the presence of a cell which has undergone L-selectin cleavage comprising the steps of:
(a) incubating said cell, or an extract thereof, with an L-selectin binding agent which is capable of selectively binding to the transmembrane domain of L-selectin; and
(b) determining whether said cell, or cellular extract, binds to said agent.

8. The method of claim 7 wherein said agent is an antibody, or antibody fragment, which is capable of binding to an epitope within the amino acid sequence of SEQ. ID No. 2.

9. A method for detecting the presence of a cell which has undergone L-selectin cleavage comprising the steps of:
(a) incubating said cell, or an extract thereof, with an agent which is capable of selectively binding to the cytoplasmic domain of L-selectin; and
(b) determining whether said cell, or cellular extract binds, to said agent.

10. The method of claim 9 wherein said agent is an antibody, or antibody fragment, which is capable of binding to an epitope within the amino acid sequence of SEQ. ID No. 3.

11. A method of identifying an agent for use in inhibiting L-selectin cleavage comprising the steps of:
a) contacting an agent with a protein containing the amino acid sequence KLDKSFSMIKEGDYN (SEQ. ID No. 1);
b) incubating the product of step (a) under condition which allows cleavage at a residue within said SEQ. ID No. 1; and
c) determining whether said agent inhibits the cleavage of said SEQ. ID NO. 1.

12. An isolated protease which cleaves within SEQ. ID No. 1.

13. The protease of claim 12, wherein said protease cleaves said SEQ. ID No. 1 after the residues "DK" and before the residues "SF".
